Europäisches Patentamt

(19) European Patent Office

Office européen des brevets

(11) Numéro de publication: **0 237 687 B1**

(12) **FASCICULE DE BREVET EUROPEEN**

(45) Date de publication du fascicule du brevet:
16.08.89

(51) Int. Cl.⁴: **G02C 13/00**

(21) Numéro de dépôt: 86400578.0

(22) Date de dépôt: 19.03.86

(54) **Appareil optique pour mesurer la position des directions visuelles principales ou autres des deux yeux et les anomalies de la vision binoculaire.**

(43) Date de publication de la demande:
23.09.87 Bulletin 87/39

(45) Mention de la délivrance du brevet:
16.08.89 Bulletin 89/33

(84) Etats contractants désignés:
AT BE CH DE FR GB IT LI LU NL SE

(56) Documents cités:
DE-A- 3 015 488
FR-A- 2 494 106
US-A- 4 208 800

(73) Titulaire: **Buget, Bernard, 16, Place de l'Alma,
F-57600 Forbach(FR)**

(72) Inventeur: **Buget, Bernard, 16 Place de l'Alma,
F-57600 Forbach(FR)**
Inventeur: **Maurice, Etienne, 7A rue de l'Eglise,
F-88360 Rupt-Sur-Moselle(FR)**

(74) Mandataire: **Bouju, André, Cabinet Bouju 38 avenue de
la Grande Armée, F-75017 Paris(FR)**

ACTORUM AG

**Description**

La présente invention concerne un appareil optique pour mesurer la position des directions visuelles principales ou autres des deux yeux et pour mesurer les anomalies de la vision binoculaire.

En absence d'anomalies de la vision binoculaire ou en vision monoculaire, la position des directions visuelles des deux yeux mesurée par l'appareil est la position des directions visuelles principales ou axes visuels.

On sait qu'il est nécessaire de connaître la position des axes visuels des deux yeux pour monter correctement dans une monture de lunette deux verres correcteurs dont les centres optiques sont situés habituellement sur les axes visuels des deux yeux.

Dans son brevet américain 4 368 958, le déposant a décrit un appareil optique adaptable devant les yeux d'un patient, qui permet de repérer avec une grande précision la position des axes visuels des deux yeux du patient.

Cet appareil comprend un support portant deux écrans mobiles en translation en matière transparente et polarisante, des moyens étant prévus pour déplacer ces deux écrans devant les yeux et pour repérer la position de ces écrans par rapport au support de l'appareil.

L'appareil ci-dessus comprend également une cible séparée du support, placée à une certaine distance devant le patient pour être visible par celui-ci. Cette cible comporte un repère central recouvert par un écran dont l'axe de polarisation est perpendiculaire à celui des écrans polarisants portés par le support. Le repère recouvert par l'écran polarisant est entouré d'une zone-repère non polarisante.

Lorsque les écrans polarisants portés par le support coupent l'axe visuel d'un oeil, le patient ne peut voir le repère de la cible avec cet oeil. Par contre, lorsqu'une arête de l'écran polarisant placé devant cet oeil est disposée tout près de cet axe visuel, le patient commence à apercevoir le repère central de la cible. Ainsi, en déplaçant successivement les écrans polarisants pour faire coïncider leurs arêtes verticales et horizontales avec l'axe visuel de chaque oeil, il est possible de déterminer avec précision la position des axes visuels des deux yeux par rapport au support de l'appareil, tout en maintenant la convergence des deux yeux sur la zone repère non polarisante.

L'appareil précité présente l'inconvénient qu'il oblige l'opticien à déplacer manuellement les écrans polarisants successivement dans deux directions perpendiculaires, ce qui rend l'utilisation de ce dispositif compliquée et sujette à des erreurs dues à des confusions.

L'un des buts de la présente invention est de remédier à cet inconvénient, en créant un appareil qui soit plus simple à utiliser et qui permette de déterminer automatiquement avec une plus grande précision la position des directions visuelles des deux yeux d'un patient.

Un autre but est de pouvoir mesurer avec ce même appareil les anomalies de la vision binoculaire, telles que le strabisme.

Actuellement, les anomalies de la vision binoculaire sont constatées en plaçant devant les yeux du patient un dispositif optique dissociant.

Le praticien demande au patient de fixer une source lumineuse placée au centre d'une croix graduée appelée croix de Madox.

En cas d'anomalie de la vision binoculaire, le point image rétinien de la source lumineuse pour un oeil, n'a pas le même point image rétinien correspondant pour l'autre oeil ; il s'en suit pour le patient une image dédoublée ou diplopie.

Comme les points rétiniens correspondants ont la même direction visuelle, on constate une déviation de la direction visuelle mesurée par le practicien en procédant de la façon suivante :
- soit par recentrement des deux images, en disposant devant l'oeil des prismes de caractéristiques optiques appropriées,
- soit en relevant directement la déviation sur la croix de Madox en demandant au patient de lire sur cette croix les graduations situes en regard de l'image déviée par rapport au centre de la croix.

Dans les deux cas s'établit un dialogue avec le patient. On comprend que cette façon d'opérer peut conduire à des erreurs dues à une mauvaise lecture des graduations par le patient.

L'appareil visé par l'invention permet de remédier à l'ensemble des lacunes de l'art antérieur exposé ci-dessus.

L'appareil optique visé par l'invention pour mesurer la position de l'axe visuel des directions visuelles principales ou autres des deux yeux et pour mesurer les anomalies de la vision binoculaire, adaptable devant les yeux du patient, conprend un support portant deux écrans mobiles en matière transparente et polarisante, des moyens pour déplacer ces deux écrans devant les deux yeux, des moyens pour repérer la position de ces écrans par rapport au support de l'appareil, une cible séparée de ce support et destinée à être placée devant le patient, cette cible comportant une zone centrale présentant un axe de polarisation perpendiculaire à celui des écrans polarisants.

Suivant l'invention, cet appareil est caractérisé en ce que chaque écran polarisant présente près de son bord latéral le plus proche de l'axe de symétrie du support passant entre les deux écrans une fente sensiblement parallèle à cet axe de symétrie et près de son bord inférieur une fente sensiblement perpendiculaire à la fente précitée et en ce que les moyens pour déplacer les deux écrans comprennent pour chaque écran un moteur de commande unique coopérant avec des moyens de transmission tels que les fentes des écrans restent parallèles à elles-même lors du déplacement des écrans et en ce sur lesdits moyens pour déplacer les deux écrans et lesdits moyens de transmission sont adaptés pour déplacer les deux écrans entre une première position dans laquelle le bord inférieur des écrans est situé au-dessus de la direction visuelle des deux yeux, une seconde position dans laquelle la fente située près du bord inférieur de chaque écran est traversée par la direction visuelle de l'oeil correspondant dirigé vers la cible, et une troisième position dans laquelle la fente située près du bord latéral de chaque écran est traversée par la direction visuelle précitée.

La cible est vue par le patient lorsque la direction visuelle passe par l'une des fentes d'un écran polarisant. Grâce à ces fentes, il est possible de repérer avec précision la position des directions visuelles par rapport à l'axe de symétrie du support passant entre les deux écrans et par rapport à un axe perpendiculaire au précédent.

Par ailleurs, étant donné que le déplacement de chaque écran polarisant est commandé par un organe de commande unique, la construction et l'utilisation de l'appareil conforme à l'invention sont considérablement simplifiées par rapport à celles de l'appareil connu décrit plus haut.

Selon une version avantageuse de l'invention, chaque écran polarisant est relié au support par deux bras parellèles articulés à l'écran et au support et l'axe d'articulation de l'un des bras au support est relié à un moteur pour faire tourner cet axe, ledit axe d'articulation étant solidaire d'un secteur denté engrénant avec un secteur denté monté sur l'axe de sortie du moteur.

Lorsque le moteur tourne, les deux bras pivotent autour de leurs deux axes solidaires du support et l'écran polarisant porté par ces deux bras se déplace devant le support de telle sorte que les fentes de l'écran restent toujours parallèles à elles-mêmes.

De préférence, le moteur commandant le déplacement des écrans polarisants est associé à des moyens permettant de repérer la position de la fente située près du bord inférieur de chaque écran par rapport à un axe de référence horizontal du support et de repérer la position de la fente située près du bord latéral de chaque écran par rapport à une position de référence perpendiculaire à la précédente et passant par l'axe de symétrie du support compris entre les deux écrans polarisants.

Le support de l'appareil conforme à l'invention peut comporter des moyens pour le fixer sur une lunette d'essai pour effectuer les examens de vision binoculaire.

Le support peut également être autonome et comporter des moyens pour le fixer directement, sans lunette intermédiaire, devant les yeux du patient, et des moyens pout fixer devant le support des lentilles de correction ou autres utiles à l'examen.

Le support de l'appareil selon l'invention peut aussi comporter des moyens pour le fixer sur la monture de lunette du patient.

D'autres particularités et avantages de l'invention apparaîtront encore dans la description ci-après.

Aux dessins annexés donnés à titre d'exemples non limitatifs :

- la figure 1 est une vue en plan montrant le devant d'un appareil conforme à l'invention fixé à la monture d'une lunete,
- la figure 2 est une vue en plan partielle de l'appareil conforme à l'invention, montrant le mécanisme de commande du déplacement d'un écran polarisant,
- la figure 3 est une vue en plan de la cible placée devant l'appareil selon l'invention,

- les figures 4 et 5 sont des vues schématiques illustrant le fonctionnement de l'appareil selon l'invention,
- la figure 6 est un schéma montrant les différentes positions d'un écran polarisant au cours d'une mesure,
- la figure 7 est une vue en perspective de trois-quart avant d'un boîtier support de cible faisant partie de l'appareil selon l'invention,
- la figure 8 est une vue schématique en coupe longitudinale du boîtier montrant son fonctionnement,
- la figure 9 est un schéma montrant l'appareil selon l'invention et sa cible associés à un microordinateur.

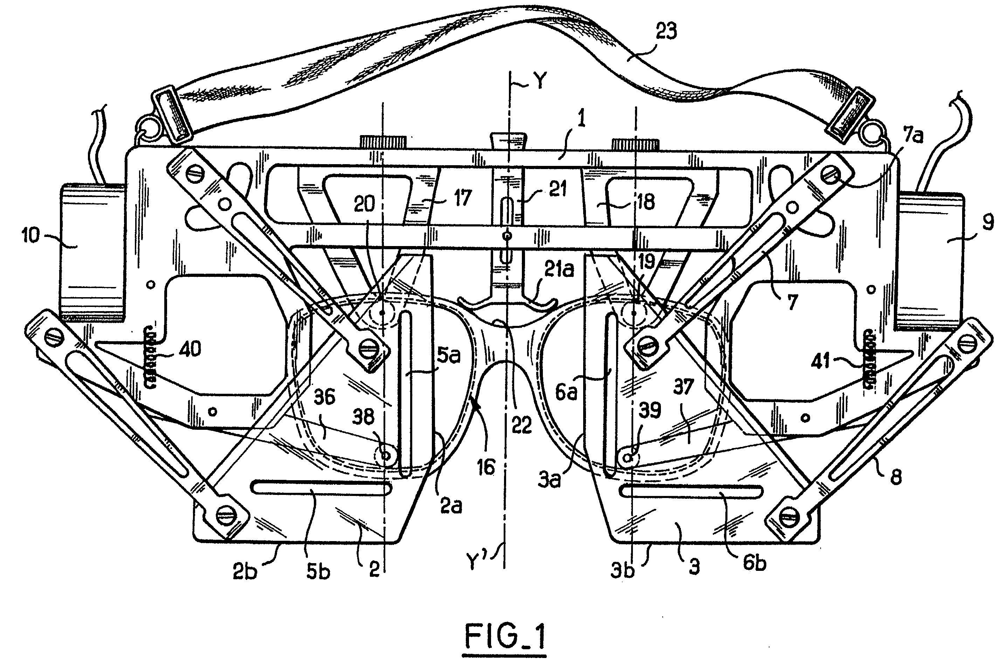

Dans la réalisation de la figure 1, l'appareil optique pour mesurer la position des directions visuelles principales ou autres des deux yeux et pour mesurer les anomalies de la vision binoculaire, adaptable devant les yeux de patient, comprend un support 1 en alliage léger ou matière plastique, portant deux écrans mobiles 2, 3 en matière transparente et polarisante, des moyens qui seront décrits plus loin pour déplacer ces deux écrans devant les deux yeux et des moyens pour repérer la position de ces écrans 2, 3 par rapport au support 1 de l'appareil.

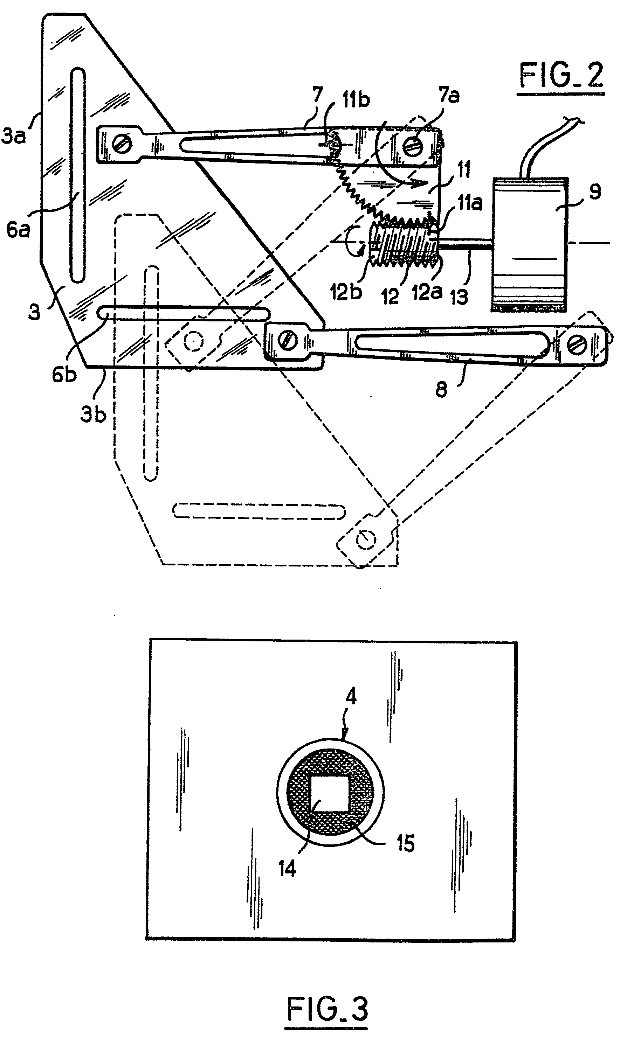

L'appareil comporte en outre une cible 4 (voir figure 3) séparée du support 1 et destinée à être placée devant le patient pour être visible par celui-ci. Cette cible présente au centre d'une zone repère 15 non polarisée, une zone 14 dont l'axe de polarisation est perpendiculaire à celui des écrans polarisants 2, 3.

Chaque écran polarisant 2, 3 présente près de son bord latéral 2a, 3a le plus proche de l'axe de symétrie Y-Y' passant entre les deux écrans 2, 3, une fente 5a, 6a sensiblement parallèle à cet axe de symétrie et près de son bord inférieur 2b, 3b une fente 5b, 6b perpendiculaire à la fente 5a, 6a. Ces fentes 5a, 6a ; 5b, 6b ont une largeur de quelques dixièmes de mm.

Les moyens pour déplacer les deux écrans 2, 3 comprennent pour chaque écran un organe de commande unique coopérant avec des moyens de transmission tels que les fentes 5a, 6a ; 5b, 6b des écrans restent parallèles à elles-mêmes lors du déplacement de ces écrans 2, 3.

Dans la réalisation représentée sur les figures 1 et 2, chaque écran polarisant 2, 3 est relié au support 1 par deux bras parallèles 7, 8 articulés à l'écran et au support 1 et l'axe d'articulation 7a est relié à un moteur 9 (ou 10) pour faire tourner cet axe.

Comme on le voit sur la figure 2, l'axe d'articulation 7a est solidaire d'un secteur denté 11 engrénant avec une vis sans fin 12 montée sur l'axe de sortie 13 du moteur 9 (ou 10). Le secteur denté comporte à chaque extrémité deux ergots 11a, 11b émergeant de la denture permettant, par un contact tangentiel à des taquets 12a, 12b montés sur l'axe de la vis sans fin, d'éviter le blocage des filets des vis sans fin en fin de course du battement des écrans polarisants. Ces butées ou autres peuvent être prévues pour limiter le déplacement des écrans 2, 3 entre une position dans laquelle (voir figure 2), les bras 7, 8 sont sensiblement horizontaux et une position (représentée en pointillés) dans laquelle ces bras 7, 8 sont inclinés vers le bas.

Dans la première position, le bord inférieur 2b ou 3b des écrans 2, 3 est situé au-dessus de la direction visuelle des deux yeux. A partir de cette première position, chaque écran 2, 3 peut être déplacé vers une seconde position dans laquelle la fente 5b, 6b située près du bord inférieur 2b, 3b chaque écran est traversée par la direction visuelle de l'oeil correspondant dirigé vers la cible 4, et une troisième position dans laquelle la fente 5a, 6a située près du bord latéral 2a, 3a de chaque écran 2, 3 est traversée par la direction visuelle précitée.

Le moteur électrique 9, 10 qui commande le déplacement des écrans 2, 3 est associé à des moyens permettant de repérer la position de la fente 5b, 6b située près du bord inférieur 2b, 3b de chaque écran 2, 3 par rapport à un axe de référence horizontal du support 1 passant par le milieu de celui-ci et de repérer la position de la fente 5a, 6a située près du bord latéral de chaque écran 2, 3 par rapport à une position de référence perpendiculaire à la précédente et passant par l'axe de symétrie Y-Y' du support 1 compris entre les deux écrans polarisés 2, 3.

De préférence, le moteur électrique 9, 10 est du type pas-à-pas et les moyens précités comprennent des moyens en eux-mêmes connus pour compter le nombre de tours de rotation du moteur 9, 10 et pour convertir ce nombre en distance de déplacement des écrans 2, 3 par rapport aux axes de référence précités. Il peut être d'un autre type avec les moyens appropriés au contrôle des déplacements.

Comme on le voit sur la figure 3, la cible 4 comporte en son centre une source lumineuse 14, par exemple de forme carrée, recouverte par un écran polarisant (non représenté) dont l'axe de polarisation est perpendiculaire à celui des écrans polarisants 2, 3 portés par le support 1. La source lumineuse 14 est entourée par une zone 15 de couleur foncée ou d'une zone lumineuse non polarisée, montée sur un dispositif pivotant. L'intensité lumineuse de la source 14 ainsi que de la zone 15 est réglable.

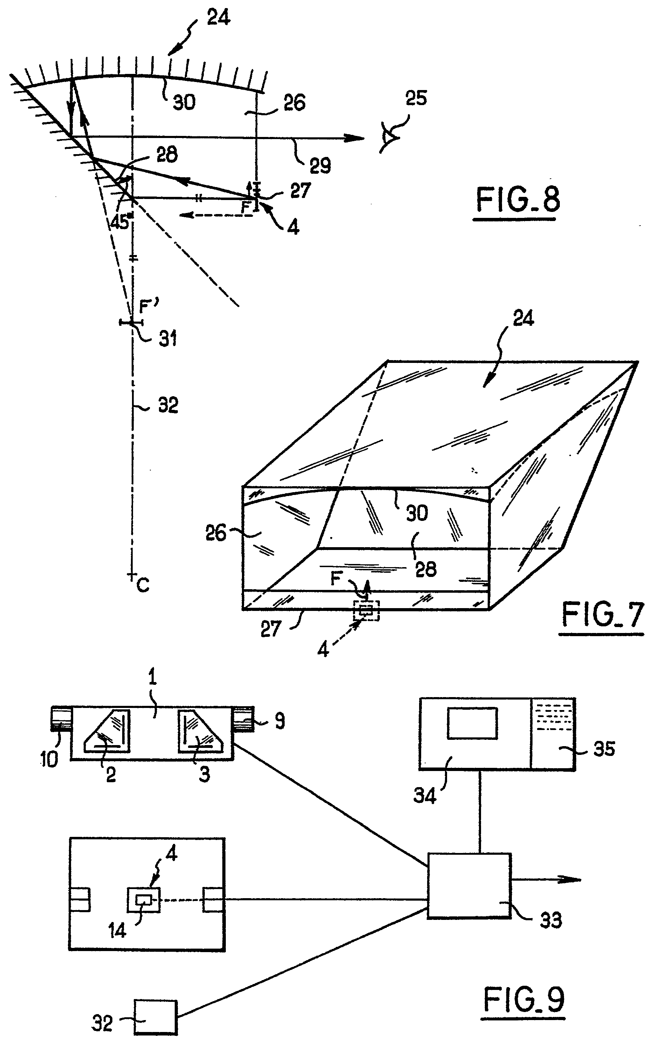

L'appareil selon l'invention comporte un commutateur 32 (voir figure 9) devant être actionné par le patient portant l'appareil pour donner des informations à un dispositif de contrôle 35 lors des modifications de sensations visuelles de l'aspect de la source 14 de la cible 4 survenant pendant le déplacement devant les yeux du patient, des écrans polarisants 2, 3 comportant les fentes 5a, 5b, 6a, 6b, entraînés par les moteurs 9 et 10 fixés au support 1.

Le dispositif de contrôle 35, par l'intermédiaire de ce commutateur 32 peut couper l'alimentation de la source lumineuse 14 dès que le patient commence à percevoir la source lumineuse, le temps que la fente traverse le champ visuel, le moteur est alors coupé par le dispositif de contrôle 35, et l'alimentation électrique de la source est rétablie automatiquement sur ordre du dispositif de contrôle alors que l'oeil se trouve caché par la partie polarisante de l'écran. Le support 1 peut également porter deux volets opaques, non représentés, rabattables sur chacun des yeux du patient, suivant la nature de l'examen effectué avec l'appareil, comme on le verra plus en détail plus loin.

Le support 1 comprend des moyens pour le fixer sur une lunette d'essai pour effectuer les examens de vision binoculaire.

Le support 1 peut également être autonome et comporter des moyens pour le fixer directement, sans lunette intermédiaire, devant les yeux du patient, et des moyens pour fixer sur le support 1 des lentilles de correction.

Dans l'example représenté sur la figure 1, le support 1 comporte des moyens pour le fixer sur la monture de lunettes 16 du patient. Ces moyens comportent des supports 17, 18 sur lesquels sont fixés des supports 19, 20 pouvant être sous forme de rondelles destinées à être introduites dans les rainures supérieures des cercles de la monture. L'écartement des supports 19, 20 est réglable par l'intermédiaire des supports 17, 18 pour s'adapter à l'écart de la monture sur la figure 1 permettant la fixation du dispositif sur des lunettes de toutes tailles. Deux leviers 36, 37 passant devant la monture dont les extrémités comportent des axes 38, 39 perpendiculaires aux leviers, recouverts d'une matière anti-dérapante viennent s'appuyer par l'intermédiaire de ressorts 40, 41 sur le bord interne de la monture. La monture se trouve bloquée entre les rondelles 19, 20 et axes 38 et 39.

Le centrage de la monture 16 par rapport au support 1 est réalisé grâce à une patte 21 fixée de façon coulissante au support 1 suivant l'axe de symétrie Y-Y' de celui-ci et dont la partie inférieure 21a prend appui dans l'encoche supérieure 22 de la monture 16.

Par ailleurs, le support 1 de l'appareil selon l'invention comporte une bande ou courroie souple 33 fixée aux extrémités opposées du support 1, destinée à être passée autour de la tête du patient de façon à assurer une parfaite stabilité de ce support 1.

On va maintenant décrire le fonctionnement de l'appareil conforme à l'invention, utilisé pour la détection et la mesure des anomalies de la vision binoculaire.

Pour cet usage, l'appareil n'étant pas destiné à être fixé sur une monture choisie par le patient, le dispositif est soit autonome, soit fixé à une monture d'essai comportant un support de lentille d'essai.

Cette mesure des anomalies de la vision binoculaire se fait en deux phases.

L'appareil conforme à l'invention permet en premier lieu de mesurer la position de l'axe visuel de chaque oeil séparé, l'autre oeil étant obturé par un volet opaque, la cible 4 étant placée à une grande distance (au moins 5 mètres) du patient ; la vision binoculaire étant interrompue, les axes visuels des deux yeux sont sensiblement parallèles.

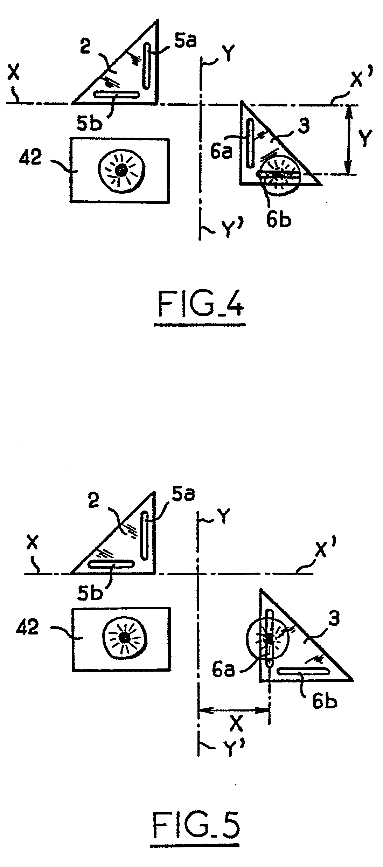

On remonte les deux écrans polarisants 2, 3 au-dessus de l'axe horizontal de référence X-X' (voir partie gauche de la figure 4) passant par le milieu du support 1. Le patient voit parfaitement des deux yeux la source lumineuse 14 de la cible 4. On descend ensuite les deux écrans polarisants 2, 3 ensemble jusqu'à ce que les bords inférieurs 2b et 3b des écrans 2, 3 coupent les axes de vision vers la source lumineuse 14 et que celle-ci ne soit plus visible par le patient.

On occulte ensuite, par le volet opaque 42, un des deux yeux pour mesurer la position de l'axe visuel de l'autre oeil. On demande alors au patient de signaler chaque apparition de la source lumineuse 14 devant l'oeil non occulté par le volet opaque.

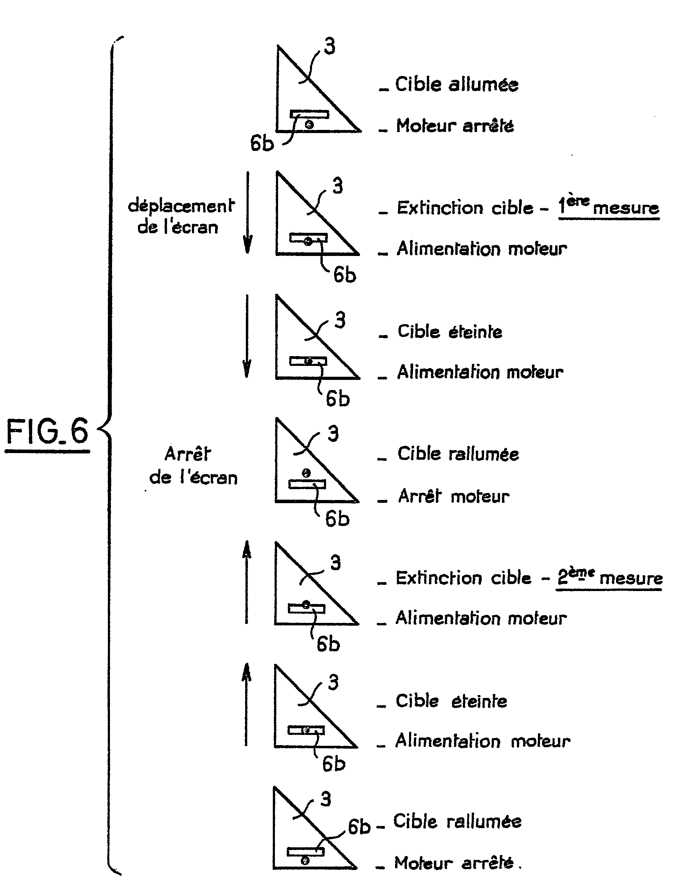

L'un des écrans polarisants, par example l'écran 3 est descendu (voir partie droite de la figure 4) jusqu'à ce que le bord inférieur de la fente horizontale 6b coupe l'axe visuel. A cet instant, la source lumineuse 14 apparaît et le patient actionne un commutateur qui, par l'intermédiaire d'un dispositif de contrôle, éteint la lumière de la source et quelque temps plus tard le moteur 9 de telle sorte que la fente soit passée devant l'oeil qui se trouve à nouveau occulté par une partie de l'écran polarisant. A l'extinction de la lumière par le commutateur, on effectue une première mesure du déplacement de l'écran 3 suivant Y-Y'. A ce moment, la source est rallumée mais invisible pour l'oeil derrière l'écran polarisant. Le mouvement du moteur est alors inversé et l'écran polarisant 3 monte jusqu'à ce que la source lumineuse 14 apparaisse à nouveau, c'est-à-dire lorsque le bord supérieur de la fente horizontale 6b coupe l'axe visuel. Le patient actionne le commutateur qui, par l'intermédiaire du dispositif de contrôle, éteint la source lumineuse 14 et arrête quelques instants plus tard le moteur, le temps nécessaire pour que l'écran polarisant se déplace devant l'oeil et l'occulte à nouveau. A l'extinction de la lumière par le commutateur, on effectue une seconde mesure du déplacement de l'écran 3 suivant Y-Y'. La moyenne des deux mesures précitées donne la position Y de l'axe visuel d'un premier oeil par rapport à l'axe X-X' de référence. Cet oeil est alors occulté par le volet opaque. Les différentes positions ci-dessus de l'écran 3 sont montrées schématiquement sur la figure 6. On procède ensuite aux mêmes mesures pour l'autre oeil.

On procède ensuite de la même façon pour déterminer pour chaque oeil la position de l'axe visuel par rapport à l'axe de référence vertical Y-Y' et on en déduit une mesure X (voir figure 5).

A partir des valeurs X et Y on peut déterminer la position des axes visuels des yeux du patient en vision monoculaire.

L'appareil conforme à l'invention permet en deuxième lieu de mesurer la position des directions visuelles de chaque oeil en vision binoculaire, le volet opaque étant relevé. On aura pris soin de disposer auparavant sur la lunette d'essai des verres dissociants utilisés habituellement dans ce type d'examen.

On peut utiliser à cet effet l'examen à la baguette de MADDOX, ou aux verres striés de BAGOLINI.

Les deux écrans polarisants 2, 3 sont remontés au-dessus de l'axe horizontal de référence X-X' puis on descend ensuite les deux écrans polarisants 2, 3 ensemble jusqu'à ce que les bords inférieurs 2b et 3b des 2, 3 coupent les directions visuelles vers la source lumineuse 14 et que celle-ci ne soit plus visible par le patient.

Le test de vision, la cible 4, étant toujours située devant le patient à 5 mètres, on effectue les mêmes mesures que précédemment à ceci près que l'écran polarisant fixe se trouve devant l'oeil qui ne fait pas l'objet de mesure.

On obtient pour chaque oeil, pour une vision à l'infini, deux positions de l'axe visuel qui permettent de déduire, s'il y a anomalie dans le cas d'une position déviée de l'axe visuel en vision binoculaire par rapport à la position de l'axe visuel en vision binoculaire interrompue par le volet opaque et de mesurer l'anomalie.

Dans une autre utilisation, le dispositif peut être appliqué sur une monture de lunette choisie par le patient et servir en absence d'anomalie de vision binoculaire à relever la position de l'axe visuel en vision de loin et vision de près. Ces mesures se font par la même méthode que précédemment sans la présence du volet opaque et des verres dissociants, en obturant l'oeil non mesuré par l'écran polarisant. Dans cette utilisation, l'axe horizontal de référence X-X' passe par les points d'intersection de la circonférence des rondelles 19, 20 avec le périmètre du gabarit de la monture en fond de rainure. La hauteur de l'axe visuel a comme origine ce point d'intersection sur l'axe médian du gabarit, le dispositif ayant été auparavant réglé à l'écart de la monture.

Les principaux avantages de l'invention sont les suivants :
- le dialogue avec le patient est minimum, de sorte que les risques d'erreurs sont très limités et la durée des mesures est beaucoup plus courte,
- les mesures sont très précises,
- l'appareil se prête à tous les examens des anomalies de la vision binoculaire.

La cible 14, au lieu d'être placée à une grande distance du patient (pour la vision de loin) ce qui n'est pas toujours possible dans un local de faible dimension, ou à une faible distance (pour la vision de près), peut être placé près du dispositif représenté sur les figures 7 et 8.

Sur ces figures, ce dispositif comprend au boîtier 24 placé à une certaine distance devant les yeux 25 du patient. Ce boîtier présente une ouverture 26 dirigée vers les yeux 25 du patient ; le bord inférieur 27 de cette ouverture 26 porte la cible 4 ; le fond du boîtier 24 opposé à l'ouverture 26 porte une surface réfléchissante plane, par exemple un miroir plan 28 formant un angle de 45° avec la direction visuelle 29 du patient. La face intérieure supérieure du boîtier 24 comporte une surface réfléchissante sphérique 30 dont la concavité est dirigée vers l'intérieur du boîtier 24. Le miroir plan 28 coupe l'axe principal 32 du miroir sphérique 30 à la moitié de la distance focale du miroir sphérique 30.

L'image 31 de la cible 4 par rapport au miroir plan 28 se trouve au foyer du miroir concave 30. Tous les rayons lumineux issus de la cible 14 après double réflexion sur le miroir plan 28 ressortent du boîtier 24 parallèles entre eux, de sorte que la cible 4 semble située à l'infini pour le patient.

Afin d'obtenir des distances d'observations différentes, notamment pour les examens de la vision rapprochée, il suffit de rapprocher la cible 4 (voir flèche F des figures 7 et 8) de l'axe principal 32 du miroir concave 30 suivant une droite perpendiculaire à cet axe, à l'intersection du miroir plan 28 avec l'axe principal du miroir concave.

Sur le schéma de la figure 9, une boîte de relais 33 relie la source lumineuse 14 de la cible 4 au commutateur 32, au support 1 comportant les moteurs électriques 9, 10 de commande des écrans polarisants 2, 3 et à un microordinateur 34 muni de moyens d'affichage 35 comportant un écran de visualisation et une imprimante, qui est capable de calculer les déplacements des écrans polarisants 2, 3, de convertir ces déplacements en positions exprimées en unités appropriées telles que dioptries ou degrés prismatiques et d'afficher ces unités.

Ainsi le microordinateur indique directement les anomalies constatées par l'appareil conforme à l'invention et des prévisions de corrections qui doivent être apportées à la vue du patient.

Bien entendu, l'invention n'est pas limitée aux exemples de réalisation que l'on vient de décrire et on peut apporter à ceux-ci de nombreuses modifications sans sortir du cadre de l'invention.

Ainsi, le support 1 de l'appareil seleon l'invention peut comporter d'autres moyens que les bras parallèles 7, 8 pour assurer que lors du déplacement des écrans polarisants 2, 3, les fentes 5a, 6a ; 5b, 6b restent parallèles à elles-mêmes, à savoir respectivement toujours verticales et toujours horizontales.

Ainsi, les bras, 7, 8 peuvent être remplacés par un levier pivotant entraîné en rotation par un moteur et solidaire d'une roue dentée engrénant une autre roue dentée fixée au levier dont l'axe est relié de façon articulée à l'écran 2 ou 3.

Dans une autre variante, un levier pivotant entraîné en rotation par un moteur comporte un disque solidaire de l'axe du moteur, ce disque présentant sur sa circonférence une articulation reliée à une bielle qui est elle-même reliée de façon articulée à un disque porté par le bras dont l'axe est relié de façon articulée à l'écran 2 ou 3.

Dans une autre variante, un levier est relié par l'une de ses extrémités à l'axe d'un moteur. L'autre extrémité de ce levier est reliée de façon articulée à l'écran polarisant 2 ou 3. Deux tiges perpendiculaires sont reliées à l'écran 2 ou 3 de façon à être respectivement parallèles à la fente verticale et à la fente horizontale de l'écran. L'extrémité de ces tiges est guidée dans des guides respectivement parallèles à la fente horizontale et à la fente verticale de l'écran, de façon que cette extrémité puisse glisser dans le guide correspondant lorsque le levier pivote et que les fentes de l'écran 2 ou 3 restent parallèles à elles-mêmes.

Bien entendu, d'autres moyens que ceux décrits brièvement ci-dessus sont encore possibles.

Par ailleurs, les volets portés par le support 1 rabattables sur chaque oeil du patient, peuvent être remplacés par des caches distincts du support 1 ou par une bande opaque disposée près du bord inférieur 2b, 3b des écrans 2, 3.

## Revendications

1. Appareil optique pour mesurer la position de l'axe visuel des directions visuelles principales ou autres des deux yeux et pour mesurer les anomalies de la vision binoculaire, cet appareil étant adaptable devant les yeux du patient et comprenant un support (1) portant deux écrans mobiles (2, 3) en matière transparente et polarisante, des moyens pour déplacer ces deux écrans (2, 3) devant les deux yeux, des moyens pour repérer la position de ces écrans par rapport au support (1) de l'appareil, une cible (4) séparée de ce support et destinée à être placée devant le patient pour être visible par celui-ci, cette cible (4) comportant une zone centrale présentant un axe de polarisation perpendiculaire à celui des écrans polarisants (2, 3), caractérisé en ce que chaque écran polarisant (2, 3) présente près de son bord latéral (2a, 3a) la plus proche de l'axe de symétrie du support (1) passant entre les deux écrans, une fente (5a, 6a) sensiblement parallèle à cet axe de symétrie, et près de son bord inférieur (2b, 3b) une fente (5b, 6b) sensiblement perpendiculaire à la fente précitée, en ce que les moyens pour déplacer les deux écrans (2, 3) comprennent pour chaque écran un moteur de commande unique (9, 10) coopérant avec des moyens de trans-mission (7, 8) tels que les fentes (5a, 6a; 5b, 6b) des écrans restent parallèles à elles-mêmes lors du déplacement des écrans, et en ce que lesdits moyens pour déplacer les deux écrans (2, 3) et lesdits moyens de transmission sont adaptée pour déplacer les deux écrans entre une première position dans laquelle le bord inférieur (2b, 3b) des écrans (2, 3) est situé au-dessus de la direction visuelle des deux yeux, une seconde position dans laquelle la fente (5b, 6b) située près du bord inférieur (2b, 3b) de chaque écran est traversée par la direction visuelle de l'oeil correspondant dirigé vers la cible (4), et une troisième position dans laquelle la fente (5a, 6a) située près du bord latéral (2a, 3a) de chaque écran est traversée par la direction visuelle précitée.

2. Appareil conforme à la revendication 1, caractérisé en ce que chaque écran polarisant (2, 3) est relié au support (1) par deux bras parallèles (7, 8) articulés à l'écran et au support et en ce que l'axe d'articulation (7a) de l'un des bras (7, 8) au support (1) est relié au moteur (9, 10) pour faire tourner cet axe.

3. Appareil conforme à la revendication 2, caractérisé en ce que ledit axe d'articulation (7a) est solidaire d'un secteur denté (11) comportant à chaque extrémité deux ergots (11a, 11b), engrénant avec une vis sans fin (12) comportant un arrêt à chaque extrémité (12a, 12b) monté sur l'axe de sortie (13) du moteur (9, 10).

4. Appareil conforme à l'une des revendications 1 à 3 et comprenant un moteur (9, 10) pour commander le déplacement de chaque écran (2, 3) polarisant entre lesdites première, seconde et troisième positions, caractérisé en ce que ce moteur (9, 10) est associé à des moyens permettant de repérer la position de la fente (5b, 6b) située près du bord inférieur (2b, 3b) de chaque écran (2, 3) par rapport à un axe horizontal (X–X') de référence passant par le milieu du support (1) ou passant par les points de référence des supports (19, 20) dont l'écartement est réglable et de repérer la position de la fente (5a, 6a) située près du bord latéral (2a, 3a) de chaque écran par rapport à l'axe de symétrie (Y-Y') du support compris entre les deux écrans polarisants (2, 3).

5. Appareil conforme à la revendication 4, caractérisé en ce que le moteur électrique (9, 10) est du type pas-à-pas et lesdits moyens comprennent des moyens pour compter le nombre de tours de rotation du moteur et pour convertir ce nombre en distance de déplacement des écrans (2, 3).

6. Appareil conforme à l'une des revendications 1 à 5, caractérisé en ce que la cible (4) est pivotante et comporte en son centre une source lumineuse (14) recouverte par un écran polarisant dont l'axe de polarisation est perpendiculaire é celui des écrans polarisants (2, 3) portés par le support (1) entourée d'une zone repère (15) non polarisée dont les intensités lumineuses sont réglables.

7. Appareil conforme à la revendication 6, caractérisé en ce qu'il comporte un commutateur (32) devant être actionné par le patient portant l'appareil pour fournir à un dispositif de contrôle (35) des informations sur la détection et les modifications des sensations lumineuses pour décider de l'alimentation électrique des moteurs électriques (9, 10) et de la source (14), et pour commander le déplacement des écrans polarisants (2, 3) portés par le support (1).

8. Appareil conforme à l'une des revendications 1 à 7, caractérisé en ce que le support (1) porte un ou deux volets opaques disposés, ou bandes opaques situées à la partie inférieure des écrans polarisants devant chacun des yeux du patient.

9. Appareil conforme à l'une des revendications 1 à 8, caractérisé en ce que le support (1) comprend des moyens pour le fixer sur une lunette d'essai pour effectuer des examens de vision binoculaire.

10. Appareil conforme à l'une des revendications 1 à 8, caractérisé en ce que le support (1) est autonome et comporte des moyens pour le fixer directement, sans lunette intermédiaire, devant les yeux du patient, et des moyens pour fixer sur le support (1) des lentilles de correction.

11. Appareil conforme à l'une des revendication 1 à 8, caractérisé en ce que le support (1) comporte des moyens (17, 18, 19, 20, 36, 37, 38, 39) pour le fixer sur la monture de lunette du patient.

12. Appareil conforme à l'une des revendications 1 à 11, caractérisé en ce qu'il comprend un boîtier (24) destiné à être placé à une certaine distance devant les yeux (25) du patient, ce boîtier présentant une ouverture (26) destinée à être dirigée vers les yeux du patient, le bord inférieur (27) de cette ouverture portant la cible (4), en ce que le fond de ce boîtier (24) opposé à l'ouverture précitée porte une surface réfléchissante plane (28) coupant l'axe principal (42) d'un miroir concave (30) à une distance égale à la moitié de la distance focale du miroir concave sous un angle de 45° dirigé vers la concavité du miroir.

13. Appareil conforme à l'une des revendications 6 à 12, caractérisé en ce que la source lumineuse (14) de la cible (4), le commutateur (32) transmetteur d'information et les moteurs électriques (9, 10) de commande des écrans polarisants (2, 3) du support (1) sont reliés par l'intermédiaire d'une boîte relais (33) à un microordinateur (35) muni de moyens d'affichage (34) et capable de calculer les déplacements des écrans polarisants (2, 3), de convertir ces déplacements en positions exprimées en unités appropriées et d'afficher les valeurs correspondantes.

**Patentansprüche**

1. Optisches Meßgerät zum Messen der Lage der Sehachse der Haupt- und anderen Blickrichtungen beider Augen und zum Messen der Anomalien des beidäugigen Sehens, wobei dieses Gerät vor den Augen des Patienten anpaßbar ist und einen Träger (1), der zwei bewegliche Schirme (2, 3) aus durchsichtigem und polarisierendem Werkstoff trägt, Mittel zum Verstellen der beiden Schirme (2, 3) vor beiden Augen, Mittel zum Bestimmen der Stellung dieser Schirme in bezug auf den Träger (1) des Gerätes, ein von diesem Träger getrenntes Zielelement (4) umfaßt, das vor dem Patienten so in Stellung bringbar ist, daß es von ihm gesehen wird, wobei das Zielelement (4) einen Mittenbereich mit einer zur Polarisierungsachse der polarisierenden Schirme (2, 3) rechtwinkligen Polarisierungsachse aufweist, dadurch gekennzeichnet, daß jeder polarisierende Schirm (2, 3) in der Nähe seines des zwischen den beiden Schirmen hindurchgehenden Symmetrieachse des Trägers (1) zunächstgelegenen Seitenrandes (2a, 3a) einen zur Symmetrieachse im wesentlichen parallelen Schlitz (5a, 6a) und in der Nähe seines unteren Randes (2b, 3b) einen zum vorgenannten Schlitz im wesentlichen rechtwinkligen Schlitz (5b, 6b) aufweist, daß die Mittel zum Verstellen der beiden Schirme (2, 3) für jeden Schirm einen einzelnen Antriebsmotor (9, 10) umfassen, der mit Übertragungsmitteln (7, 8) zusammenwirkt, die so sind, daß die Schlitze (5a, 6a; 5b, 6b) der Schirme beim Verstellen der Schirme zueinander parallel bleiben und daß die genannten Mittel zum Verstellen der beiden Schirme (2, 3) und die genannten Übertragungsmittel die beiden Schirme zwischen einer ersten Stellung, in welcher der untere Rand (2b, 3b) der Schirme (2, 3) oberhalb der Blickrichtung der beiden Augen angeordnet ist, einer zweiten Stellung, in welcher die Blickrichtung des entsprechenden, gegen das Zielelement (4) gerichteten Auges durch den in der Nähe des unteren Randes (2b, 3b) jedes Schirms angeordneten Schlitz (5b, 6b) hindurchgeht, und einer dritten Stellung zu verstellen vermögen, in welcher die vorgenannte Blickrichtung durch den in der Nähe des Seitenrandes (2a, 3a) jedes Schirms angeordneten Schlitz (5a, 6a) hindurchgeht.

2. Gerät nach Anspruch 1, dadurch gekennzeichnet, daß jeder polarisierende Schirm (2, 3) mit dem Träger (1) durch zwei am Schirm und am Träger schwenkbar gelagerte parallele Arme (7, 8) verbunden ist und daß die Achse (7a) für die schwenkbare Lagerung eines der Arme (7, 8) am Träger (1) zu ihrem Drehantrieb mit dem Motor (9, 10) verbunden ist.

3. Gerät nach Anspruch 2, dadurch gekennzeichnet, daß die genannte Schwenkachse (7a) mit einem verzahnten Sektor (11) fest verbunden ist, der an jedem Ende zwei Vorsprünge (11a, 11b) aufweist und mit einer Schnecke (12) kämmt, die an jedem Ende (12a, 12b) einen Anschlag aufweist und auf der Abtriebswelle (13) des Motors (9, 10) angeordnet ist.

4. Gerät nach einem der Ansprüche 1 bis 3 und mit einem Motor (9, 10) für den Verstellantrieb jedes polarisierenden Schirms (2, 3) zwischen der genannten ersten, zweiten und dritten Stellungen, dadurch gekennzeichnet, daß dieser Motor (9, 10) Mitteln zugeordnet ist, welche die Ermittlung der Stellung der in der Nähe des unteren Randes (2b, 3b) jedes Schirms (2, 3) angeordneten Schlitzes (5b, 6b) in bezug auf eine waagerechte Bezugsachse (X–X'), die durch die Mitte des Trägers (1) oder durch die Bezugspunkte der Träger (19, 20) geht, deren Abstand voneinander einstellbar ist, und die Ermittlung der Stellung der in der Nähe des Seitenrandes (2a, 3a) jedes Schirms angeordneten Schlitzes (5a, 6a) in bezug auf die zwischen den beiden polarisierenden Schirmen (2, 3) gelegene Symmetrieachse (Y–Y') des Trägers ermöglichen.

5. Gerät nach Anspruch 4, dadurch gekennzeichnet, daß der elektrische Motor (9, 10) ein Schrittmotor ist und die genannten Mittel Mittel zum Zählen der Anzahl Umdrehungen des Motors und zum Umwandeln dieser Zahl in Verstellweg der Schirme (2, 3) umfassen.

6. Gerät nach einem der Ansprüche 1 bis 5, dadurch gekennzeichnet, daß das Prüfelement (4) schwenkbar ist und in seiner Mitte eine Lichtquelle (14) aufweist, die mit einem polarisierenden Schirm bedeckt ist, dessen Polarisierungsachse zu jener der vom Träger (1) getragenen polarisierenden Schirme (2, 3) rechtwinklig ist, und von einer nichtpolarisierten Merkzeichenzone (15) umschlossen ist, deren Lichtstärken einstellbar sind.

7. Gerät nach Anspruch 6, dadurch gekennzeichnet, daß es einen vom das Gerät tragenden Patienten zu betätigenden Schalter (32) aufweist, um einer Steuervorrichtung (35) Informationen über die Erfassung und die Veränderungen der Lichtempfindungen zu liefern zur Entscheidung über die elektrische Speisung der elektrischen Motoren (9, 10) und der Quelle (14) und zum Betätigen der Verstellung der vom Träger (1) getragenen polarisierenden Schirme (2, 3).

8. Gerät nach einem der Ansprüche 1 bis 7, dadurch gekennzeichnet, daß der Träger (1) ein oder zwei undurchsichtige Klappen oder Bänder trägt, die im unteren Abschnitt der polarisierenden Schirme vor jedem der Augen des Patienten angeordnet bzw. gelegen sind.

9. Gerät nach einem der Ansprüche 1 bis 8, dadurch gekennzeichnet, daß der Träger (1) Mittel aufweist, um ihn zur Durchführung von Tests für beidäugiges Sehen an einer Prüfbrille zu befestigen.

10. Gerät nach einem der Ansprüche 1 bis 8, dadurch gekennzeichnet, daß der Träger (1) selbsttragend ist und Mittel zu seiner direkten Befestigung vor den Augen des Patienten ohne Zwischenbrille und Mittel zur Befestigung von Korrekturlinsen auf dem Träger (1) aufweist.

11. Gerät nach einem der Ansprüche 1 bis 8, dadurch gekennzeichnet, daß der Träger (1) Mittel (17, 18, 19, 20, 36, 37, 38, 39) zu seiner Befestigung an der Brillenfassung des Patienten aufweist.

12. Gerät nach einem der Ansprüche 1 bis 11, dadurch gekennzeichnet, daß es ein Gehäuse (24) umfaßt, das in einer bestimmten Entfernung vor den Augen (25) des Patienten in Stellung bringbar ist, eine gegen die Augen des Patienten zu richtende Öffnung (26) aufweist, deren unterer Rand (27) das Zielelement (4) trägt, daß die der vorgenannten Öffnung gegenüberliegende Rückwand dieses Gehäuses (24) eine ebene Reflexionsfläche (28) trägt, welche die Hauptachse (42) eines Hohlspiegels (30) in einer Entfernung, die der halben Brennweite des Hohlspiegels gleich ist, unter einem Winkel von 45° gegen die Konkavität des Spiegels schneidet.

13. Gerät nach einem der Ansprüche 6 bis 12, dadurch gekennzeichnet, daß die Lichtquelle (14) des Zielelementes (4), der Schalter (32) zur Informationsübertragung und die elektrischen Antriebsmotoren (9, 10) für die polarisierenden Schirme (2, 3) des Trägers (1) durch einen Relaiskasten (33) an einen mit einer Anzeigeeinrichtung (34) versehenen Kleinrechner (35) angeschlossen sind, der die Verstellungen der polarisierenden Schirme (2, 3) zu berechnen, diese Bewegungen in Stellungen, ausgedrückt in zweckdienlichen Einheiten, umzuwandeln und die entsprechenden Werte anzuzeigen vermag.

**Claims**

1. An optical device for measuring the position of the visual axis of the main or other visual directions of both eyes and for measuring anomalies of binocular vision, the device being adapted to be fitted in front of the patient's eyes and comprising: a support (1) carrying two mobile transparent polarizing screens (2, 3); means for moving the same in front of both eyes; means for detecting the position of the screens relatively to the support (1); a target (4) separate therefrom and adapted to be placed before, and to be visible by, the patient and having a central zone whose polarization axis is perpendicular to that of the polarizing screens (2, 3), characterised in that each polarizing screen (2, 3) is formed, near its side edge (2a, 3a) nearest the axis of symmetry of the support (1) passing between the two screens, with a slot (5a, 6a) substantially parallel to the latter axis and near its bottom edge (2b, 3b) with a slot (5b, 6b) substantially perpendicular to the first-mentioned slot (5a, 6a); the means for moving the two screens (2, 3) comprise for each screen a single drive motor (9, 10) co-operating with transmission means (7, 8) such that the screen slots (5a, 6a; 5b, 6b) of the screens stay parallel to themselves during movements of the screens; and the means for moving the two screens (2, 3) and the transmission means are adapted to move the two screens between a first position, in which the bottom edge (2b, 3b) of the screens (2, 3) is disposed above the visual direction of both eyes, a second position, in which the visual direction of the corresponding eye directed towards the target (4) extends through the slot (5b, 6b) near the bottom edge (2b, 3b) of each screen, and a third position, in which the said visual direction extends through the slot (5a, 6a) near the side edge (2a, 3a) of each screen.

2. A device according to claim 1, characterised in that each polarizing screen (2, 3) is connected to the support (1) by two parallel arms (7, 8) articulated to the screen and to the support and the connecting pivot (7a) of one of the arms (7, 8) to the support (1) is connected to the motor (9, 10) for rotation thereby.

3. A device according to claim 2, characterised in that the pivot pin (7a) is rigidly secured to a toothed sector (11) having at each end two pins (11a, 11b) meshing with a worm (12) comprising at each end (12a, 12b) a stop and disposed on the output shaft (13) of the motor (9, 10).

4. A device according to any of claims 1–3 and comprising a motor (9, 10) for moving each polarizing screen (2, 3) between the said first, second and third positions, characterised in that the motor (9, 10) is associated with means for detecting the position of the slot (5b, 6b) near the bottom edge (2b, 3b) of each screen (2, 3) relatively to a horizontal reference axis (X–X') passing through the centre of the support (1) or through the reference points of the supports (19, 20), the spacing of which is adjustable, and detecting the position of the slot (5a, 6a) near the side edge (2a, 3a) of each screen relatively to the axis of symmetry (Y–Y') of the support between the two polarizing screens (2, 3).

5. A device according to claim 4, characterised in that the electric motor (9, 10) is of the stepping kind and the means comprise means for counting the number of revolutions of the motor and converting such number into the distance travelled by the screens (2, 3).

6. A device according to any of claims 1–5, characterised in that the target (4) is pivoted and has at its centre a light source (14) covered by a polarizing screen whose polarization axis is perpendicular to that of the polarizing screens (2, 3) on the support (1), the light source (14) being surrounded by an unpolarized detection zone (15) whose light intensities are adjustable.

7. A device according to claim 6, characterised in that it comprises a selector (32) operable by the wearer of the device to supply a control facility (35) with information on the detection and modifications of light sensations to decide th electrical energization of the electric motors (9, 10) and source (14) and to control the movement of the polarizing screens (2, 3) on the support (1).

8. A device according to any of claims 1–7, characterised in that the support (1) has one or two opaque shutters disposed at, or opaque strips situated at, the bottom part of the polarizing screens before each of the patient's eyes.

9. A device according to any one of claims 1–8, characterised in that the support (1) comprises means for securing it to a test spectacle frame for making examinations of binocular vision.

10. A device according to any one of claims 1–8, characterised in that the support (1) is independent and has means for being secured directly without intermediate frame before the patient's eyes and means for securing correcting lenses to the support (1).

11. A device according to any of claims 1–8, characterised in that the support (1) has means (17–20, 36–39) for securing it to the rim of the patient's spectacle.

12. A device according to any of claims 1–11, characterised in that it comprises a casing (24) adapted to be placed at a distance before the patient's eyes (25) and formed with an aperture (26) directable theretowards, the bottom edge (27) of the aperture (26) carrying the target (4), and the casing member remote from the aperture (26) has a plane reflecting surface (28) intersecting the main axis (42) of a concave mirror (30) at a distance equal to half the focal length of the concave mirror at an angle of 45° directed towards the mirror concavity.

13. A device according to any of claim 6–12, characterised in that the light source (14) of the target (4), the information-transmitting selector (32) and the electric motors (9, 10) for moving the polarizing screens (2, 3) are connected by way of a relay box (33) to a microcomputer (35) having display means (34) and adapted to calculate the movements of the polarizing screens (2, 3), convert such movements into positions expressed in appropriate units and display the corresponding values.

FIG_1

FIG_2

FIG_3

EP 0 237 687 B1

FIG_4

FIG_5

EP 0 237 687 B1

FIG.6

**déplacement de l'écran**

- Cible allumée
- Moteur arrêté

- Extinction cible - $1^{ère}$ mesure
- Alimentation moteur

- Cible éteinte
- Alimentation moteur

**Arrêt de l'écran**

- Cible rallumée
- Arrêt moteur

- Extinction cible - $2^{ème}$ mesure
- Alimentation moteur

- Cible éteinte
- Alimentation moteur

- Cible rallumée
- Moteur arrêté.

_FIG. 8_

_FIG. 7_

_FIG. 9_